Europäisches Patentamt

(19) European Patent Office  (11) Publication number: **0 050 061**

Office européen des brevets  **B2**

(12) **NEW EUROPEAN PATENT SPECIFICATION**

(45) Date of publication of the new patent specification: **20.06.90**

(21) Application number: **81401468.4**

(22) Date of filing: **22.09.81**

(51) Int. Cl.⁵: **A 61 K 35/16,** A 61 K 37/00, A 61 L 2/18, B 01 F 17/00

---

(54) Method of reducing undesirable activities of biological and pharmaceutical products.

---

(30) Priority: **06.10.80 US 194264**
**06.10.80 US 194263**

(43) Date of publication of application:
**21.04.82 Bulletin 82/16**

(45) Publication of the grant of the patent:
**11.12.85 Bulletin 85/50**

(45) Mention of the opposition decision:
**20.06.90 Bulletin 90/25**

(84) Designated Contracting States:
**AT BE CH DE FR GB IT LI NL SE**

(56) References cited:
**EP-A-0 005 864**
**US-A-4 102 996**
**US-A-4 105 650**

**CHEMICAL ABSTRACTS, vol. 80, no. 25, June 24th 1974, page 251, 2nd column, abstract no. 143780s, COLUMBUS OHIO (US), L. BERTOK and L.T. KOCSAR: "Biological properties of surfactant treated endotoxin in rats"**

(73) Proprietor: **New York Blood Center, Inc.**
**310 East 67 Street**
**New York, New York 10021 (US)**

(72) Inventor: **Shanbrom, Edward**
**2252 Liane Lane**
**Santa Ana, California 92705 (US)**

(74) Representative: **Bernasconi, Jean et al**
**CABINET LEMOINE ET BERNASCONI 13,**
**Boulevard des Batignolles**
**F-75008 Paris (FR)**

(56) References cited:
**CHEMICAL ABSTRACTS, vol. 74, no. 17, April 26th 1971, page 263, 2nd column, abstract no. 85878t, COLUMBUS OHIO (US), J.P. JILKINS: "Decreased shock lethality in rats with surfactant treated endotoxin"**

**CHEMICAL ABSTRACTS, vol. 70, no. 1, January 6th 1969, page 23, 1st column, abstract no. 291r, COLUMBUS OHIO (US), J.A. RUDBACH et al.: "Reaction of endotoxin and surfactants. III. Effect of sodium lauryl sulfate on the structure and pyrogenicity of endotoxin"**

Courier Press, Leamington Spa, England.

(56) References cited:

CHEMICAL ABSTRACTS, vol. 84, no. 16, April
19th 1976, page 405, 1st column, abstract no.
111640n, COLUMBUS OHIO (US), SATOSHI
FUNAKOSHI: "Homogeneous hepatitis B
antigen"

FILKINS, Proc. Soc. Exp. Biol. Med. 1971, 136(2),
pp. 466-8

E. RIBI et al, J. of Bacteriology, (1966), 92(5), pp.
1493 et seq.

RUDBACH, Can.J. Microbiol. 1968, 14 (11), pp.
1173-8

Annals N.Y. Acad. Sciences 133 (1966), pp. 629
et seq.

F. Sokol et al., Virology 38, pp. 651-665,(1969)
J. Skelly et al., J. Gen. Virol. 44, pp. 679-689,
(1979)

S. Asculai et al., Antimicrobic Agents and
Chemotherapy, (13), No. 4, pp. 686-690

B. Horowitz, Transfusions, 25, pp. 516-522,
(1985)

**Description**

This invention relates to a method of reducing or suppressing undesirable activities like pyrogenicity, hepatitis infectivity and clotting activation of biological and pharmaceutical products, including plasma, plasma derivatives and products thereof, or of products for treating them, like sterile filters and chromatographic materials.

In the development and production of biological and pharmaceutical products and particularly various proteinaceous substances used for biomedical and therapeutic purposes, the problem of contamination with pyrogens (endotoxins) is ever present. For certain products, like products from human plasma, transmission of viral hepatitis, and activation of the coagulation enzymes also make major problems.

Pyrogens are lipopolysaccharides (LPS) derived from the outer cell wall of gram-negative bacteria. They are toxic materials which are also known as endotoxins to distinguish them from toxic substances synthesized and excreted by the intact bacterium. Pyrogens have numerous biologic activities which include the production of fever, activation of clotting mechanisms and induction of shock. Consequently, it is essential that pyrogenic substances be removed and that the causative bacteria be rendered innocuous by sterilization or other such treatment of the final biological or pharmaceutical product.

Prior methods for such inactivation or destruction of pyrogens comprise extensive treatment with heat, acid or alkali, filtration of insoluble pyrogens, or removal by adsorption with gels, ion-exchange resins and various other such adsorbent materials. Most of these methods are burdensome, time consuming, or destructive of the protein due to the rigorousness of the treatment.

Further background on the properties and effects of pyrogens can be had by reference to a paper by Elizabeth Work entitled "Production, Chemistry and Properties of Bacterial Pyrogens and Endotoxins" in "Pyrogens and Fever", Ciba Foundation Symposium, 1971, pp. 23—47, edited by Wolstenholme and Birch, published by Churchill Livingstone; and a paper by D. C. Morrison and R. J. Ulevitch entitled "The Effects of Bacterial Endotoxins on Host Mediation Systems"; in Amer. J. Pathol. 93 (2), 527—601 (1978).

It has been proposed to reduce S. enterides endotoxins toxicity by adding certain amphiphiles to preparations of lipopolysaccharide from such bacteria (J. P. Filknis, Proc. Soc. Exp. Biol. Med. 1971, 136(2), pages 466—8). Where inactivation of the endotoxins resulted in vitro, endotoxin shock in the rat was merely ameliorated but not avoided.

J. A. Rudbach et al. (Can. J. Microbiol 1968, 14 (11) pages 1173—8) showed that E. coli and S. enterides endotoxins have a reduced pyrogenicity when dissociated in Na deoxycholate. Nevertheless the endotoxins are not inactivated in vitro to an extent which would avoid endotoxic shock.

Similar teaching is given by E. Ribi et al. (J. of Bacteriology, Nov. 1966, 92 (5), pages 1493 to 1509) which show that Na deoxycholate lowers pyrogenicity of endotoxins when a small amount of proteins is added.

In Annals N.Y. Academy of Sciences, 133, pages 629 to 643, (1966) J. A. Rudbach et al., confirm that Na deoxycholate is able to split the endotoxin polymer into smaller non-toxic elements and that some plasma proteins prevent the reassociation, but in a reversible way so that the inactivation is reversible.

It is well-known that plasma and products made from plasma may transmit hepatitis. Until recently, interest has focused primarily on hepatitis B antigen (HB₅Ag) as the offending agent and attempts at eliminating this agent have led to widespread screening of all plasma used in transfusing by a variety of laboratory procedures. While such laboratory screening has apparently decreased the incidence of hepatitis B in patients receiving whole blood transfusions, there has not been significant improvement in the incidence of the disease transmitted from plasma products. Attempts to remove the virus by various adsorption procedures or precipitation techniques, e.g. with polyethylene glycol, have not proven to eliminate infectivity. There is some evidence that the combination of ultraviolet light and β-propionolactone may be helpful in inactivating the virus in certain plasma products. However, there is some apprehension that β-propionolactone has carcinogenic properties.

While the development of screening tests for hepatitis B has been of limited value in reducing transmission of the disease, the identification of this virus (as well as the hepatitis A virus) has led to the recognition of a third virus which is apparently responsible for the majority of cases of hepatitis transmitted by blood plasma derivatives. This virus is referred to as "non-A, non-B hepatitis". Tests for this virus are not yet commercially availabe for wide-spread screening. This virus closely resembles hepatitis B virus but is antigenically distinguishable. Both hepatitis B virus and non-A, non-B hepatitis virus appear to have similar structural characteristics and exist as particles containing a DNA core and a lipoprotein membrane.

The Chemical Abstracts (1976), 84: 111 640 h states that Hepatitis B antigen isolated from blood serum can be dissociated by surfactants into subunits but these latter are rather highly active.

Attempts to prevent activation of clotting enzymes have centered around the addition of classic anticoagulating chemicals such as citrate, EDTA and heparin. While such measures are partially effective, none of these anti-coagulants are effective at the early stage of the clotting sequence which is triggered by activation of Factor XII. Activation of clotting factors may still occur because coagulation may be instigated by the presence of phospholipids from blood platelets. Virtually all blood fractions collected today contain thromboplastic-like substances or phospholipids from platelets and platelet derivatives which activate coagulation. Administration of therapeutic blood fractions which contain these phospholipid particles may be potentially dangerous because of their ability to induce unwanted intravascular coagulation.

The US patent specification 4 105 650 discloses use of a surfactant (Pluronic®) as a preservative agent for AHF in the plasma cryoprecipitate recovery route of AHF. This preservative property for AHF and possibly related proteins is deemed to be inferred from the close relation of Pluronic® to PEG (polyethylene glycol) and this specification does not suggest that Pluronic® would have any activity in reducing or suppressing undesirable activities like pyrogenicity, HBAg or clotting activation.

In accordance with the present invention the method of reducing or eliminating substances causing undesirable activities selected from pyrogenicity, viral infectivity and activation of clotting factors in biological or pharmaceutical products, comprising the step of adding an amphiphile, is characterized by treatment of said products by prolonged contact of at least about 30 minutes with a solution or suspension of from 0.25% to 10% by weight of a non denaturing amphiphile and then, removing said amphiphile with at least the most important part of the pyrogenicity causing undesirable substance(s), if any, from said products.

The term "non-denaturing" means non-protein-denaturing. The subject treatment of the products causes irreversible destruction of endotoxins and if any destroys thromboplastic-like coagulation activating substances and substantially reduces the infectivity of hepatitis viruses (B and non-A, non-B). These effects are produced without substantially altering the activity of product constituents, for example plasma proteins.

As used herein, the term "amphiphile" is meant to define a substance containing both hydrophilic water-soluble and hydrophobic water-insoluble groups. Amphiphiles are generally classified into various groups and frequently into the anionic, cationic, ampholytic and non-ionic surfactants. The following are well-known commercially available amphiphiles:

Anionic

Sulphated oxyethylated alkylphenol (Triton W-30)®;
Sulphated lauryl ether alcohol;
Sodium dodecylbenzenesulfonate (Nacconol NR)®;
Sodium 2-sulfoethyl oleate (Igepon A)®;
Sodium N-methyl-N-oleylethanol sulfonate (Igepon T)®;
Sodium dodecylsulfate;
Sodium cholate;
Sodium deoxycholate;
Sodium dodecylsulfonate;
Sodium dodecyl-N-sarconisate.

Cationic

Dodecyldimethylbenzylammonium chloride (Triton K-60)®;
Oxyethylated amines (Ethomeen)®;
Cetyltrimethylammonium bromide;
Tetradecylammonium bromide;
Dodecylpyrimidinium chloride;
Hexadecyltrimethylammonium chloride.

Ampholytic

Dodecyl β-alanine;
N-dodecylaminoethanesulfonic acid;
Palmitoyllysolecithin;
Dodecyl-N-betaine.

Nonionic

Ethylene oxide-propylene oxide condensates (Pluronic® block copolymers) such as described in U.S. Patent 2,674,619; Oxyethylated alkylphenol (Triton X-100)®; Partial esters of $C_{12-22}$ fatty acids (e.g. lauric, palmitic, stearic and oleic acids); and hexitol anhydrides (e.g. hexitans and hexides) (Spans) such as described in U.S. Patents 2,232,820; 2,232,821; 2,303,432; Polyoxyethylated derivatives of said partial esters made by adding polyoxyethylene chains to the nonesterified hydroxyls (Tweens®, e.g. Tween 80® or Polysorbate 80®) such as described in U.S. Patent 2,380,166; Polyoxyethylene partial fatty acid esters (Myrj 45)®; Polyoxyethylene fatty alcohol ethers (Brij)®.

The nonionic surfactants are preferred amphiphiles for use in this invention. The most preferred amphiphiles are the nonionic surfactants having a high water solubility and selected from the group consisting of substances having the general formula $RC_6H_4(OC_2H_4)_nOH$ wherein R is octyl or nonyl and n is at least 3. A most preferred substance of the foregoing general formula is octyl phenoxy polyethoxy ethanol. Surfactants of the latter type are available commercially from Rohm & Haas Co. under the trademarks "Triton X", e.g., Triton X-100, Triton X-165, Triton X-205, Triton X-305 and Triton X-405. Another such nonionic surfactant is nonyl phenoxy polyethoxy ethanol which is available commercially under the trademark "Triton N-100".

4

Another preferred group of amphiphiles are the salts of bile acids such as sodium cholate and sodium deoxycholate.

Treatment of the biological and pharmaceutical products with the amphiphile can be carried out at any stage in the production sequence. Preferably, for depyrogenation, the treatment is carried out following the last step at which contamination with pyrogens is likely to occur. In those instances where pyrogen contamination occurs at a production stage following a previous depyrogenation, it may be necessary to subject the product to a further depyrogenation treatment in accordance with the method of this invention. The method of the invention also is useful for reworking biological and pharmaceutical products that have become pyrogenic in a normal production run.

The period of time during which the biological and pharmaceutical products are contacted with the amphiphile should be sufficient to depyrogenate the product is of at least about 30 minutes and may be extended to about four hours at a temperature of from about 4°C to about 37°C and is adequate to provide the desired depyrogenation.

Testing for the presence of pyrogens and to ensure adequate depyrogenation can be carried out by the standard qualitative fever response test in rabbits for pyrogens or by more recently developed Limulus lysate (amebocytes) assay procedures for pyrogens (LAL tests). The latter tests are based on gelling of a pyrogenic preparation in the presence of the lysate of the amebocytes of the horseshoe crab (*Limulus polyphemus*). See e.g. U.S. Patent 4,096,091 for a typical LAL test.

Treatment of the plasma protein product with the amphiphile can be carried out at any stage in the production process for destroying endotoxin or infectivity of hepatitis virus and preventing of clotting activation. The amphiphile can be added to the starting material or it can be added at some later step in the production sequence.

In the case of therapeutic plasma protein products it will generally be desirable to remove the amphiphile following the prolonged contact with the plasma protein product. In the case of non-therapeutic plasma protein products it will generally be unnecessary to remove the amphiphile. For example, blood plasma and blood fractions for administration to human patients would be subjected to a step for ultimate removal of the amphiphile following the prolonged contact whereas clinical sera for diagnostic purposes generally would not require such removal. Removal of the amphiphile can be carried out by various precipitation steps in which the plasma proteins are precipitated while the amphiphile remains dissolved or suspended in the supernatant. Conventional plasma protein precipitants can be used for this purpose such as, e.g., polyethylene glycol, Pluronic® polymers, glycine, ammonium sulfate, alcohol and rivanol.

The period of time during which the plasma protein product is contacted with the amphiphile should be sufficient to cause irreversible destruction of endotoxins and/or inactivation of hepatitis viruses is of at least about 30 minutes and may be extended to about 4 hours at a temperature of from about one degree C to about 50°C and is adequate to provide the desired destruction of endotoxins and inactivation of hepatitis viruses. It will be appreciated, however, that for non-therapeutic and non-human administration of plasma protein products, the destruction of endotoxins is not critical although the destruction of hepatitis infectivity is important to avoid spread of the virus to the laboratory workers.

The actual contact of the biological and pharmaceutical products with the amphiphile can be carried out by washing the product with a solution or suspension of the amphiphile or by immersing or soaking the product in such solution or suspension or by admixing the product with such solution or suspension.

Although certain amphiphiles such as sodium deoxycholate have been reported heretofore as able to dissociate or disaggregate endotoxins, the disaggregation has been described as reversible in the presence of the amphiphile tested. These amphiphiles thus have not been previously suggested as able to produce irreversible disaggregation of endotoxins such as to make them practical for the treatment of plasma protein products which are to be used for human administration. See, e.g., the paper by Elizabeth Work, cited hereinbefore. In accordance with the present invention, the desired plasma protein product is precipitated with protein precipitents, as described above, after treatment with the amphiphile to destroy the endotoxins followed by separation and removal of the amphiphile in the supernatant. In such case it has been found that the Lipid A or most active portion of the endotoxin which normally is insoluble in water, remains with the amphiphile in the supernatant. The disaggregation is irreversible and treatment previously not believed to be useful is now rendered practical.

The present invention is applicable to any biological or pharmaceutical product which because of its intended use in humans or administration to humans for biomedical or therapeutic purposes should be free of pyrogens and otherwise sterile. It is particularly adapted for depyrogenating those products which cannot be adequately depyrogenated by heat or pH adjustments. Many proteinaceous products fall in the latter category due to the potential denaturation or destruction of the active material which can be caused by the prolonged rigorous treatment with heat, acid or alkali. For treatment of such proteinaceous products the amphiphile should be non-protein-denaturing.

Examples of biological and pharmaceutical products which can be depyrogenated in accordance with the present invention are:

Blood and blood fractions such as antihemophilic factor A (AHF, Factor VIII); prothrombin complex (Factors II, VII, IX and X); gamma globulin; albumin.

Biological and pharmaceutical products derived from animal origin, e.g., insulin and other hormones, enzymes;

Biological products involved in or derived from tissue culture techniques;

Vaccines, including substances derived from animal and microbial sources;

Biological products derived from human or animal placenta;

Pharmaceutical and drug products in which the crude drug product is produced by fermentation of microorganisms which generate endotoxins;

Pharmaceutical and drug products which are processed in equipment having a residue of microbial contamination;

Products prepared by recombinant DNA or gene-splicing techniques and in which the products are elaborated by genetically engineered microorganisms such as in *E. coli,* e.g., strain K12, or *Bacillus subtilis* and the like. Examples of such products and the applicable technology can be had by reference to UK Patent Application No. 2,018,778; European Patent Applications 6694 and 9930; Belgian Patent 867,424; U.S. Patent 4,190,495; and German Offenlegungsschriften 2,848,051; 2,848,052; 2,848,053; 2,923,927; and 2,940,525.

Further, although certain other amphiphiles such as Triton X-100®, Tween 80®, Nonidet NP-40®, and sodium dodecylsulfate have been reported heretofore as able to dissociate or disaggregate the hepatitis virus antigen, the disaggregation has been described for the purpose of merely dispersing the antigen or for purifying the antigen for its use in preparing vaccines. See, e.g., U.S. Patents 4,118,748; 4,118,749; 4,164,565; and a paper by Johnson et al., *J. Lab. Clin. Med. 88*(1), 91—101 (1976). As distinguished from the above, treatment of the plasma protein product with the amphiphile under the conditions herein described according to the present invention is to substantially reduce the infectivity of hepatitis viruses in such products whereby the plasma protein product is itself improved.

Testing for the presence of hepatitis virus and/or to determine its destruction following treatment of the plasma protein product according to the present invention can be carried out by various conventional laboratory test methods such as reversed passive hemagglutination assays, counter-electrophoresis (CEP) and the more recently developed radioimmunoassay (RIA) procedures. Solid phase RIA procedures for hepatitis antigen as described in U.S. Patents 3,867,517 and 4,012,494 and commercially available from Abbott Laboratories under the trademark "Ausria" are illustrative of suitable methods. It should be understood, however, that the determination of the presence of hepatitis antigen is limited by the sensitivity of these tests and that negative test results do not rule out the possible presence of the antigen in extremely low, non-detectable levels.

For reducing or suppressing hepatitis infectivity and/or clotting activation, the present invention is applicable to all types of plasma protein products for therapeutic as well as non-therapeutic uses. Examples of such products are:

Blood and blood fractions such as antihemophilic factor A (AHF, Factor VIII); prothrombin complex (FActors II, VII, IX and X); gamma globulin; albumin; and the like;

Clinical diagnostic control sera containing human or animal (e.g., porcine or bovine) plasma protein components (e.g., albumin); and

Placenta plasma proteins.

The following examples will further illustrate the invention although it will be understood that the invention is not limited to these specific examples.

Example 1

Whole human blood plasma was diluted 1:2 with pyrogen-free water and then spiked with ten micrograms of *E. coli* endotoxin. Triton X-100®, was added to a concentration of 2% and the mixture was then incubated at 37°C for one hour. Three dilutions of the treated mixture were then made and the dilutions were tested for pyrogenicity by the Limulus Lysate (amebocytes) assay (LAL test) with the following results:

| Blood dilutions | Endotoxin amount | % Triton® X-100 | LAL Reaction |
|---|---|---|---|
| 1:10 | 1 microgram | 0.2 | Negative |
| 1:100 | 0.1 microgram | 0.02 | Negative |
| 1:1000 | 0.01 microgram | 0.002 | Negative |

Example 2

A commercially produced vial (lyophilized) of prothrombin complex (25 units of Factor IX per ml when reconstituted) was reconstituted with ten ml of sterile water. Triton X-100® was added to a concentration of 2% and the mixture was incubated ninety minutes at ambient temperature (ca. 20—22°C). To nine ml of the above mixture was added 6.0 ml of a 50% solution of polyethylene glycol 4000. The pH was adjusted to 5.7 and the mixture was cooled to below 5°C to precipitate the protein. The mixture was centrifuged and the precipitate was resuspended in sterile water. The treated product was tested for pyrogenicity by the LAL test and compared with the original untreated sample and a control which consisted of the aforesaid precipitate of the treated sample to which 40 picograms of endotoxin were added. The following results were observed:

| | LAL Reaction | | | |
|---|---|---|---|---|
| Dilutions | 1:10 | 1:20 | 1:40 | 1:80 |
| Original sample untreated | pos.×2 | pos.×2 | pos.×2 | pos.×2 |
| Precipitate of sample with 40 picograms Endotoxin | neg.* | pos.×2 | pos.×2 | pos.×2 |
| Triton X-100® treated precipitate | neg.×2 | neg.×2 | neg.×2 | neg.×2 |

Example 3

Similar depyrogenation of endotoxin-contaminated albumin is obtained by treatment with 2% Triton X-100® as in Examples 1 and 2, above, or with 2% Polysorbate® 80.

Example 4

Similar depyrogenation of endotoxin-contaminated fibrinogen is obtained by treatment with 2% Triton X-100® as in Examples 1 and 2, above, or with 2% Polysorbate 80®.

Example 5

Clinical diagnostic control sera were analyzed for their various components both before and after treatment with Triton X-100® at a concentration of 2% to destroy endotoxins and substantially reduce hepatitis virus infectivity in accordance with the present invention. The following results on the untreated and treated product show that the Triton X-100® treatment has not substantially altered the activity of the various components in the control sera.

| Analyte | Untreated (control) | Treated |
|---|---|---|
| Calcium (mg/dl) | 14.3 | 13.4 |
| Phosphorus (mg/dl) | 3.5 | 3.4 |
| Glucose (mg/dl) | 235 | 219 |
| BUN (mg/dl) | 53 | 50 |
| Uric acid (mg/dl) | 8.1 | 7.9 |
| Cholesterol (mg/dl) | 186 | 172 |
| Total protein (gm/dl) | 8.4 | 8.2 |
| Albumin (gm/dl) | 4.7 | 4.4 |
| Tot. bilirubin (mg/dl) | 3.9 | 3.8 |
| Alk. phosphatase (IU/L) | 162 | 145 |
| LDH (IU/L) | 325 | 412 |
| SGOT (IU/L) | 105 | 103 |
| Sodium (meq/l) | 146 | 139 |
| Potassium (meq/l) | 5.7 | 5.3 |
| Chloride (meq/l) | 111 | 107 |
| Carbon dioxide (meq/l) | 28 | 26 |
| Creatinine (mq/dl) | 3.4 | 3.1 |
| SGPT (IU/L) | 118 | 107 |
| Uric acid (mg/dl) (Hycel Mark X) | 8.8 | 8.1 |
| Uric acid (mg/dl) (12/60, 6/60) | 8.5 | 8.3 |

The aforesaid analysis was made on Technicon 12/60, 6/60 AutoAnalyzer equipment except as noted for uric acid on a Hycel Mark X.

## Example 6

Human growth hormone derived by recombinant DNA techniques from *E. coli* strain K12, was treated with 3% Triton X-100®, for two hours and reprecipitated with ammonium sulfate. The pyrogen level in the product was reduced from 2.2 nanograms/ml in the untreated product to less than 10 picograms/ml in the treated product.

## Example 7

Various therapeutic blood plasma protein products are treated by prolonged contact with a solution of 2% Triton X-100® and subsequently separated from the Triton X-100® by precipitation as follows:

(a) for the production of AHF, Triton X-100® is added to whole blood plasma prior to the conventional cryo-precipitation step at freezing temperatures of −20°C to −80°C. The Triton X-100® remains in the supernatant while AHF is concentrated in the cryoprecipitate.

(b) For the production of gamma globulin, Triton X-100® is added to whole blood plasma at the beginning of the Cohn fractionation procedure with cold alcohol according to Method 6. The Triton X-100® remains in the supernatant while gamma globulin is precipitated in Cohn fraction II+III.

(c) For the production of albumin, Triton X-100® is added to the supernatant after the removal of Cohn fraction II+III in the Cohn fractionation procedure with cold alcohol according to Method 6. The Triton X-100® remains in the supernatant while albumin is precipitated principally in Cohn fraction V.

(d) For the reworking of pyrogenic albumin, albumin powder is resuspended and Triton X-100® is added to the suspension. Albumin is then reprecipitated with a protein precipitant such as polyethylene glycol, alcohol or ammonium sulfate and Triton X-100® remains in the supernatant.

Example 8

Various non-therapeutic blood plasma protein products such as fetal bovine serum, bovine albumin and bovine serum which are commonly used as growth media in tissue culture procedures are treated by prolonged contact with 2% Triton X-100® as in the previous examples. The Triton X-100® is removed in the supernatant after precipitation of all proteins as in Example 7 and resuspension in appropriate milieu, e.g., balanced salt solution, such as Hank's BSS.

Example 9

When 2% Triton X-100® is added to platelet-poor plasma (double spun plasma) the non-activated partial thromboplastin time (PTT) becomes indefinitely prolonged instead of the usual 200 seconds. This evidences the anticoagulant activity of Triton X-100® in the plasma.

(a) Whole blood collected in 2% Triton X-100® does not clot for over 12 hours.

(b) Whole blood collected in 2% sodium cholate does not clot in over 24 hours.

Example 10

Samples of blood sera and plasma obtained for laboratory analysis are placed in test tubes containing 2% Triton X-100® and allowed to incubate for 30—120 minutes to inactive potential hepatitis virus.

**Claims**

1. The method of reducing or eliminating contaminating substances causing undesirable activities selected from pyrogenicity, viral infectivity and activation or clotting factors, in biological or pharmaceutical products, comprising the step of adding an amphiphile characterized by treatment of said products by prolonged contact or at least about 30 minutes with a solution or suspension of from 0.25% to 10% by weight of a non-denaturing amphiphile and then, removing said amphiphile with at least the most important part of the pyrogenicity causing undesirable substance(s), if any, from said products.

2. The method of claim 1, characterized by the treatment of proteins containing biological or pharmaceutical products, including plasma, plasma derivatives and proteins, hormones, enzymes, products extracted from placenta, vaccines, products derived from tissue, culture techniques, pharmaceutical and drug products produced by fermentation of micro organisms, products prepared by recombinant DNA or gene-splicing techniques.

3. The method of claim 1, characterized by the treatment of non-protein-containing biological or pharmaceutical products, including vaccines, pharmaceutical and drug products having a residue of microbial contamination, intravenous solutions.

4. The method of claim 1, characterized by the treatment of biomedical implants.

5. The method of any one of claims 1 to 4, characterized in that the amphiphile is a bile acid salt selected from sodium cholate and sodium deoxycholate.

6. The method of any one of claims 1 to 4, characterized in that the amphiphile is a nonionic surfactant.

7. The method of claim 6, characterized in that the nonionic surfactant is selected from the polyoxyethylated derivatives of partial esters of $C_{12-22}$ fatty acids and hexitol anhydrides.

8. The method of claim 7, characterized in that the nonionic surfactant is poly(oxyethylene(20)) sorbitan monooleate.

9. The method of claim 6, characterized in that the nonionic surfactant is selected from substances having the general formula $RC_6H_4(OC_2H_4)_nOH$ wherein R is octyl or nonyl and n is at least 3.

10. The method of claim 9, characterized in that the nonionic surfactant is octyl phenoxyl polyethoxyl ethanol.

11. The method of claim 8, characterized in that the concentration of the surfactant is about 2—3%.

12. The method of any one of claims 1 to 3 and 5 to 11, characterized in that the amphiphile is removed from said product by at least one precipitation step.

13. The method of claim 12 for treating a therapeutic blood plasma fraction, characterized in that the amphiphile is removed from said product following said prolonged contact by precipitation of said plasma fraction with a plasma protein precipitant while said amphiphile remains dissolved or suspended in the supernatant.

14. The method of any one of claims 1 to 13, characterized by about four hours contact of the amphiphile with said products.

**Revendications**

1. Procédé de réduction ou d'élimination, dans des produits biologiques ou pharmaceutiques, de substances contaminantes entraînant des activités indésirables parmi l'effet pyrogène, l'activité infectieuse virale et l'activation des facteurs l'aggrégation, comprenant l'étape d'adjonction d'un amphiphile, caractérisé par le traitement desdits produits par contact prolongé d'au moins environ 30 minutes avec une solution ou suspension de 0,25% à 10% en poids d'un amphiphile non dénaturant et, ensuite, la séparation dudit amphiphile avec au moins la partie la plus importante de(s) substance(s) indésirable(s) entrainant l'effet pyrogène, si présente(s), d'avec lesdits produits.

2. Procédé selon la revendication 1, caractérisé par le traitement de produits biologiques ou pharmaceutiques contenant des protéines, y compris de plasma, de protéines et dérivés du plasma, hormones, enzymes, produits extraits du placenta, vaccins, produits dérivés de techniques de culture tissulaire, produits pharmaceutiques et médicaments produits par fermentation de micro-organismes, produits préparés pare recombinaison d'ADN ou techniques de manipulations génétiques.

3. Procédé selon la revendication 1, caractérisé par le traitement de produits biologiques ou pharmaceutiques ne contenant pas de protéines, y compris de vaccins, produits pharmaceutiques et médicaments possédant un résidu de contamination microbienne, solutions intraveineuses.

4. Procédé selon la revendication 1, caractérisé par le traitement d'implants biomédicaux.

5. Procédé selon l'une quelconque des revendications 1 à 4, caractérisé en ce que l'amphiphile est un sel d'acide biliaire choisi parmi le cholate de sodium et le désoxycholate de sodium.

6. Procédé selon l'une quelconque des revendications 1 à 4, caractérisé en ce que l'amphiphile est un surfactant non ionique.

7. Procédé selon la revendication 6, caractérisé en ce que le surfactant non ionique est choisi parmi les dérivés polyoxyéthylènes d'esters partiels d'acides gras $C_{12-22}$ et d'anhydrides d'hexitols.

8. Procédé selon la revendication 7, caractérisé en ce que le surfactant non ionique est un monooléate de poly(oxyéthylène(20)) sorbitan.

9. Procédé selon la revendication 6, caractérisé en ce que le surfactant non ionique est choisi parmi les substances ayant la formule générale $RC_6H_4(OC_2H_4)_nOH$ où R est octyle ou nonyle et n est au moins égal à 3.

10. Procédé selon la revendication 9, caractérisé en ce que le surfactant non ionique est l'octylphénoxyl polyéthoxyl éthanol.

11. Procédé selon la revendication 8, caractérisé en ce que le concentration du surfactant est d'environ 2—3%.

12. Procédé selon l'une quelconque des revendications 1 à 3 et 5 à 11, caractérisé en ce que l'amphiphile est séparé d'avec ledit produit par au moins une étape de précipitation.

13. Procédé selon la revendication 12, pour traiter une fraction de plasma sanguin thérapeutique, caractérisé en ce que l'amphiphile est séparé d'avec ledit produit à la suite dudit contact prolongé par précipitation de ladite fraction de plasma avec un agent précipitant de protéine de plasma tandis que ledit amphiphile reste dissous un suspendu dans le surnageant.

14. Procédé selon l'une quelconque des revendications 1 à 13, caractérisé par un contact d'environ 4 heures de l'amphiphile avec lesdits produits.

**Patentansprüche**

1. Verfahren zur Verringerung oder Beseitigung von verseuchenden Substanzen die unerwünschte Wirkungen bewirken zwischen Pyrogenizität, Virus-Infectiosität und Aktivierung der Coagulationsfactoren, in biologischen oder pharmazeutischen Produkten, das die Stufe der Zugabe eines Amphiphils umfasst, gekennzeichnet durch die Behandlung dieser Produkte durch einen verlängerten Kontakt von mindestens etwa 30 Minuten mit einer Losung oder Suspension von 0.25 bis 10 Gew. % eines nicht-denaturierenden Amphiphils und danach der Entfernung des Amphiphils mit mindestens des wichtigen Teiles der Pyrogenizität bewirkenden ungewünschten Substanz(en), falls anwesend, aus diesen Produkten.

2. Verfahren nach Anspruch 1, gekennzeichnet durch die Behandlung von proteinhaltigen biologischen oder pharmazeutischen Produkten einschließlich Plasma, Plasmaderivaten und Proteinen, Hormonen, Enzymen, Produkten, die aus Placenta extrahiert wurden, Vakzinen, Produkten, die abgeleitet wurden von Gewebekulturverfahren pharmazeutischen und Medikamentprodukten, die durch Fermentation von Mikroorganismen hergestellt wurden, Prudukten, die durch Deoxyribonucleinsäure-Rekombinat oder Gen-Spleißungs-Verfahren hergestellt wurden.

3. Verfahren nach Anspruch 1, gekennzeichnet durch die Behandlung von nicht-proteinhaltigen biologischen oder pharmazeutischen Produkten, einschließlich Vakzinen, pharmazeutischen und Medikamentprodukten mit einem Rückstand von mikrobiologischen Verunreinigungen, intravenösen Lösungen.

4. Verfahren nach Anspruch 1, gekennzeichnet durch die Behandlung von biomedizinischen Implantaten.

5. Verfahren nach einem der Ansprüche 1—4, dadurch gekennzeichnet, daß das Amphiphil ein Gallensäuresalz ist, ausgewählt aus Natriumcholat und Natriumdeoxycholat.

6. Verfahren nach einem der Ansprüche 1—4, dadurch gekennzeichnet, daß das Amphiphil ein nichtionisches Tensid ist.

7. Verfahren nach Anspruch 6, dadurch gekennzeichnet, daß das nicht-ionische Tensid ausgewählt ist aus den polyoxyethylierten Derivaten der partiellen Ester von $C_{12-22}$-Fettsäuren und Hexitanhydriden.

8. Verfahren nach Anspruch 7, dadurch gekennzeichnet, daß das nicht-ionische Tensid Poly(oxy-ethylen(20))sorbitanmonooleat ist.

9. Verfahren nach Anspruch 6, dadurch gekennzeichnet, daß das nicht-ionische Tensid ausgewählt ist aus den Substanzen, die die allgemeine Formel $RC_6H_4(OC_2H_4)_nOH$ haben, worin R eine Octyl- oder Nonylgruppe darstellt und n mindestens 3 ist.

10. Verfahren nach Anspruch 9, dadurch gekennzeichnet, daß das nicht-ionische Tensid Octylphenoxylpolyethoxylethanol ist.

11. Verfahren nach Anspruch 8, dadurch gekennzeichnet, daß die Konzentration des Tensids etwa 2—3% beträgt.

12. Verfahren nach einem der Ansprüche 1—3 und 5—10, dadurch gekennzeichnet, daß das Amphiphil aus dem Produkt durch mindestens einen Fällungsschritt entfernt wird.

13. Verfahren nach Anspruch 12 zur Behandlung einer therapeutischen Blutplasmafraktion, dadurch gekennzeichnet, daß das Amphiphil aus diesem Produkt entfernt wird, nach dem verlängerten Kontakt, durch Fällung der Plasmafraktion mit einem Plasma-Protein-Fällungsmittel, wobei das Amphiphil in dem Obenstehenden gelöst oder suspendiert bleibt.

14. Verfahren nach einem der Ansprüche 1—13, gekennzeichnet, durch einen etwa 4-stündigen Kontakt des Amphiphils mit diesen Produkten.